(19) Europäisches Patentamt
European Patent Office
Office européen des brevets

(11) Veröffentlichungsnummer: **0 195 975 A2**

(12) **EUROPÄISCHE PATENTANMELDUNG**

(21) Anmeldenummer: **86103201.9**

(22) Anmeldetag: **10.03.86**

(51) Int. Cl.4: **C07C 49/637**, C07C 45/27, C07C 45/29, C11B 9/00, A61K 7/46, A23L 1/00

(30) Priorität: **25.03.85 CH 1304/85**

(43) Veröffentlichungstag der Anmeldung: **01.10.86 Patentblatt 86/40**

(84) Benannte Vertragsstaaten: **CH DE FR GB LI NL**

(71) Anmelder: **L. GIVAUDAN & CIE Société Anonyme**
**CH-1214 Vernier-Genève(CH)**

(72) Erfinder: **Bachmann, Jean-Pierre**
**Seestrasse 28**
**CH-8806 Bäch(CH)**
Erfinder: **Pesaro, Mario, Dr.**
**Wiesliacher 55**
**CH-8053 Zürich(CH)**

(74) Vertreter: **Urech, Peter, Dr. et al**
**Grenzacherstrasse 124 Postfach 3255**
**CH-4002 Basel(CH)**

(54) **Bicyclisches Keton, Verfahren zu dessen Herstellung und Riech- und/oder Geschmackstoffkompositionen mit einem Gehalt an diesem bicyclischen Keton.**

(57) Die Erfindung betrifft einen neuen Riech-und/oder Geschmackstoff, nämlich das 3,5,5,6,8,8-Hexamethyl-1,2,3,4,5,6,7,8-octahydro-2-naphthalenon und ein Verfahren zu dessen Herstellung.

Die Erfindung betrifft auch Riech-und/oder Geschmackstoffkomposition mit einem Gehalt an dem neuen Keton und schliesslich die Verwendung des neuen Ketons als Riech und/oder Geschmackstoff.

EP 0 195 975 A2

Bicyclisches Keton, Verfahren zu dessen Herstellung und Riech-und/oder Geschmackstoffkompositionen mit einem Gehalt an diesem bicyclischen Keton

Die Erfindung betrifft einen neuen Riech-und/oder Geschmackstoff. Es handelt sich debei um das 3,5,5,6,8,8-Hexamethyl-1,2,3,4,5,6,7,8-octahydro-2-naphthalenon, also um die Verbindung der Formel

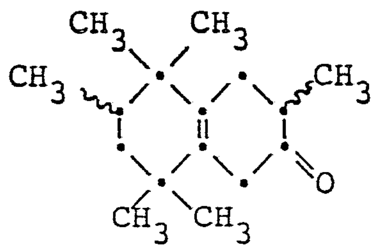

I

Die Verbindung I kann als Gemisch der zwei möglichen Racemate

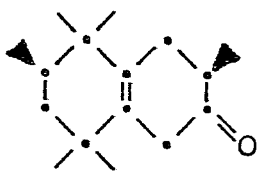

und

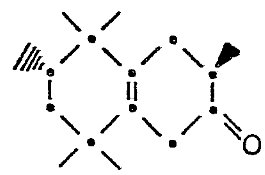

Ia

Ib

oder als eines dieser Racemate vorliegen.

Die Erfindung betrifft ferner ein Verfahren zur Herstellung von I.

Dieses Verfahren ist dadurch gekennzeichnet, dass man 1,1,3,4,4,6-Hexamethyl-1,2,3,4,5,8-hexahydronaphthalin hydroboriert und das Hydroborierungsprodukt zum 3,5,5,6,8,8-Hexamethyl-1,2,3,4,5,6,7,8-octahydro-2-naphthalenon oxydiert.

Bei der Hydroborierung von 1,1,3,4,4,6-Hexamethyl-1,2,3,4,5,8-hexahydronaphthalin entsteht durch Anlagerung von Boran an die 6,7-Doppelbindung des Ausgangsmaterials der Formel

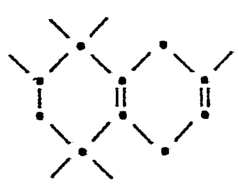

II

ein Organoboran.

Dieses Organoboran kann nun entweder direkt zum Keton I, oder über eine alkoholische Zwischenstufe, nämlich die Verbindung der Formel

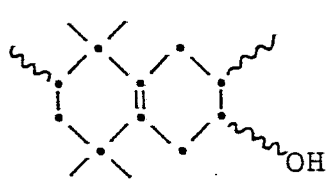

III

zu I oxidiert werden.

Die untenstehende Tabelle 1 gibt eine detaillierte Uebersicht über die Herstellung der neuen Verbindung I gemäss den beiden Verfahrensvarianten sowie über einen zweckmässigen Zugang zum entsprechenden Ausgangsmaterial II:

Tabelle 1

| Verfahrensschritt | Reaktionstyp | Mittel | Lösungsmittel | Temperatur |
|---|---|---|---|---|
| II → Organoboran | Hydroborierung (1) | $B_2H_6$ | Aether, z.B. Diäthyläther, Tetrahydrofuran, Diglym, etc. | |
| Organoboran → I | Oxidation | $Cr_2O_7^{2-}/H^+$ ($H_2SO_4$) | | -20 - +30°C, insb. Raumtemperatur |
| Organoboran → III | Oxidation | $H_2O_2/OH^-$ (NaOH) | | 0-50°C, insb. 0-25°C |
| III → I | Oxidation Alkohol → Keton (2) | insb. $Cr^{6+}$-Salze z.B. Jones-Reagens (4) | Aceton | 0-50°C, insb. 0 - 25°C |
| Herstellung von II | Reduktion (3) | (3) | (3) | (3) |

(1) siehe z.B. Brown, Hydroboration, W.A. Benjamin Inc. N.Y. 1962; Boran-Lösungen in Tetrahydrofuran sind handelsüblich, man kann sie aber auch leicht herstellen, z.B. durch Reaktion von Natriumborhydrid mit Bortrifluoridätherat.

(2) siehe z.B. "Oxidation", Vol. 1, Marcal Bekker Inc. N.Y. (1969) edited by R.L. Augustine, Seiten 56 -81.

(3) zweckmässigerweise Reduktion von 1,1,3,4,4,6-Hexamethyl-1,2,3,4-tetrahydronaphthalin, siehe z.B. die Europäische Patentpublikation Nr. 115 274 vom 8. August 1984

(4) zweckmässigerweise hergestellt durch Lösen von Chromtrioxid in konzentrierter Schwefelsäure und Verdünnen

mit Wasser: J. Chem. Soc., 457 (1953).

Die Verbindung der Formel I zeichnet sich durch kräftige, diffusive und sehr natürlich-warme Noten in Richtung Moschus, mit fruchtigen, sandelholzartigen und animalischen Geruchsaspekten aus.

Die Erfindung betrifft demgemäss auch die Verwendung der Verbindung I als Riech-und/oder Geschmackstoff.

Die Verbindung der Formel I eignet sich aufgrund ihrer natürlichen Geruchsnoten insbesondere zur Modifizierung von bekannten Kompositionen. Hervorgehoben werden soll nun insbesondere ihre ausserordentliche Geruchsstärke: Der Geruschsschwellenwert beträgt 0,13 ng/l; der Geruchswert 179,800. Bezüglich Definition von Geruchswert und Geruchsschwellenwert siehe z.B. Ulrich A. Huber, Seifen - Oele -Fette -Wachse 110, Nr. 15 (1984) 448-451. Die entsprechenden Werte -unter denselben Bedingungen gemessen -für das bekannte, strukturell ähnliche 6,7,-Dihydro-1,1,2,3,3-pentamethyl-5-(4H)-indanon sind andererseits 2.5 ng/l und 35,500.

Die Verbindung I verbindet sich mit zahlreichen bekannten Riechstoffingredientien natürlichen oder synthetischen Ursprungs, wobei die Palette der natürlichen Rohstoffe sowohl leicht-als auch mittel-und schwer-flüchtige Komponenten, und diejenige der Synthetika Vertreter aus praktisch allen Stoffklassen umfassen kann, wie dies aus der folgenden Zusammenstellung ersichtlich ist:

-Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,

-Alkohole, wie Farnesol, Geraniol, Linalool, Nerol, Phenyläthylalkohol, Rhodinol, Zimtalkohol,

-Aldehyde, wie Citral, Helional R, $\alpha$-Hexylzimtaldehyd, Hydroxycitronellal, Lilial R (p-tert. Butyl-$\alpha$-methyl-dihydrozimtaldehyd), Methylnonylacetaldehyd,

-Ketone, wie Allyljonon, $\alpha$-Jonon, $\beta$-Jonon, Isoraldein - (Isomethyl-$\alpha$-ionon), Methyljonon,

-Ester, wie Allyl-phenoxyacetat, Benzyl-salicylat, Cinnamylpropionat, Citronellyl-acetat, Citronellyl-äthoxalat (Citronellyl . O -CO -CO . $OC_2H_5$), Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat,

-Lactone, wie $\gamma$-Undecalacton,

-verschiedene, in der Parfümerie oft benützte Komponenten, wie Ketonmoschus, Indol, p-Menthan-8-thiol-3-on, Methyleugenol.

Bemerkenswert ist ferner die Art und Weise, wie die Verbindung I die Geruchsnoten bekannter Kompositionen abrundet und harmonisiert, ohne aber in unangenehmer Weise zu dominieren. So unterstreicht sie z.B. in Parfümbasen mit Tee-und Grün-Charakter die weiche und blumige Note, und in Rosenbasen wird der gesuchte Charakter der schweren und süssen bulgarischen Rose unterstrichen.

In Fruchtbasen kann die Verbindung I mit Erfolg zum Erzielen eines samtig-weichen, natürlich-süssen und abrundenden Effekts eingesetzt werden.

Die Verbindung der Formel I (bzw. deren Gemische) lässt sich in weiten Grenzen einsetzen, die beispielsweise von 0,1 (Detergentien) -5% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,2 und 2%. Die mit I hergestellten Kompositionen lassen sich für alle Arten von parfümierten Verbrauschsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindung I kann demgemäss bei der Herstellung von Kompositionen und -wie obige Zusammenstellung zeigt -unter Verwendung einer breiten Palette bekannter Riechstoffe bzw. Riechstoffgemische, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1874 hervorgehend.

Die neue Verbindung der Formel I ist ebenfalls vorzüglich geeignet zur Verwendung in Fruchtaromen verschiedenster Art, insbesondere aber auch zur Aromatisierung von Tabak.

Als Geschmackstoff kann die Verbindung I beispielsweise zur Erzeugung bzw. Verbesserung, Verstärkung, Steigerung oder Modifizierung von Fruchtaromen verschiedenster Art, z.B. Brombeer-oder Aprikosenaromen verwendet werden. Als Anwendungsgebiet dieser Aromen kommen beispielsweise Nahrungsmittel (Joghurt, Süsswaren, etc.), Genussmittel (Tee, Tabak, etc.) und Getränke (Limonade etc.) in Frage.

Die ausgeprägten geschmacklichen Qualitäten der Verbindung I ermöglicht die Verwendung als Aromastoff in geringen Konzentrationen. Eine geeignete Dosierung umfasst beispielsweise den Bereich von 0.01 ppm -100 ppm, vorzugsweise den Bereich von 0.01 ppm -20 ppm, im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50 -500 ppm.

Die Verbindung I kann auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoff kompositionen verstanden, die sich auf an sich bekannte Art verdünnen bzw. in essbaren Materialien verteilen lassen. Sie enthalten beispielsweise etwa 0,1 -5, insbesondere 0,2 -3 Gew.% an Verbindung I. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung enthalten oder können der einschlägigen Literatur entnommen werden, siehe z.B.

E. Ziegler, Die natürlichen und künstlichen Aromen, Dr. Alfred Hüthig Verlag GmbH, Heidelberg, 1982.

H.B. Heath, Flavor Technology: Profiles, Products, Applications, Avi Publishing Company, Inc. Westport, Connecticut, 1978.

G. Fenaroli, Fenaroli's Handbook of Flavor Ingredients, Second Edition, Volume 2, CRC-Press, Inc. Cleveland, Ohio 1975, oder

J.Merory, Food Flavorings, Composition, Manufacture and Use, Second Edition, Avi Publishing Company, Inc., Westport, Connecticut, 1968.

Für die Herstellung der üblichen Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminosin-5'-monophosphat (IMP), Dinatriumguanosin-5-phosphat (GMP); oder spezielle Aromastoffe, Wasser, Aethanol, Propylenglykol, Glycerin.

Beispiel 1

Eine Lösung von 215,4 g des Produktes der Reduktion von 1,1,3,4,4,6-Hexamethyl-1,2,3,4-tetrahydronaphthalin mit Lithium/Aethanol in Methylamin (enthaltend gemäss gaschromatographischer Analyse 58% 1,1,3,4,4,6-Hexamethyl-1,2,3,4,5,8-hexahydronaphthalin) in 580 ml Tetrahydrofuran wird mit 16,23 g fein pulverisiertem Natriumborhydrid versetzt. Innert 15 Minuten wird eine Lösung von 72,5 ml Bortrifluoridethylaetherat (ca. 48% $BF_3$) in 116 ml Tetrahydrofuran zugetropft und das Gemisch 2 Stunden bei Raumtemperatur gerührt. Nach Kühlen auf + 5°C werden innert 10 Minuten 96 ml Wasser, darauf innert 10 Minuten 193 ml 5 n NaOH und zuletzt innert 45 Minuten 193 ml 30-prozentiges Wasserstoffperoxid zugetropft.

Das Reaktionsgemisch wird noch 2,5 Stunden bei 45°C gerührt, dann in Hexan aufgenommen und mit Wasser neutral gewaschen. Nach dem Trocknen der organischen Phase über $MgSO_4$ und Einengen am Rotationsverdampfer verbleiben 234,1 g farbloses Oel, dessen Hauptprodukte die diastereomeren 3,5,5,6,8,8-Hexamethyl-1,2,3,4,5,6,7,8-octahydro-2-naphthalenole III sind.

Dieses Oel (234,1 g) wird in 1,06 l Aceton gelöst; bei 0°C werden 291 ml Jones-Reagens so zugetropft, dass die Innentemperatur +5°C nicht übersteigt (ca. 60 Minuten). Nach weiteren 15 Minuten nimmt man das Reaktionsgemisch in Hexan auf und wäscht es mit Wasser neutral; die wässrigen Phasen werden mit Hexan extrahiert und die organische Phase über $MgSO_4$ getrocknet. Es verbleiben nach dem Einengen 208,8 rohes Oel, welche zunächst über eine kleine Vigreuxkolonne schnell-destilliert werden:

Fraktionen:

75 -96°C/0,07 mbar : 34,5 g;

98 -108°C/0.07 mbar : 146,9 g;

Rückstand : 22,7 g.

Die Fraktion mit dem Siedepunkt 98 -108°C/0,07 mbar wird über eine 25 cm-Widmerkolonne fein-destilliert:

Fraktionen:

71 -107°C/0,05 mbar, $n_D^{20}$ 1,4892-1,4971 : 53,6 g;

107 -113°C/0,05 mbar, $n_D^{20}$ 1,4971-1,4980 : 74,3 g;

113 -109°C/0,05 mbar, $n_D^{20}$ 1,5000 : 5.8 g;

Rückstand : 12,2 g

Die Fraktionen mit dem Siedepunkt 107°C - 113°C/0,05 mbar (74,3 g) stellen die olfaktisch gute Qualität von 3,5,5,6,8,8-Hexamethyl-1,2,3,4,5,6,7,8-octahydro-2-naphthalenon dar (Gemisch von 2 Racematen).

$n_D^{20}$ = 1,4975

IR ($CHCl_3$): 1705 $cm^{-1}$

$^1$H-NMR (400 MHz, $CDCl_3$): δ(ppm) 0,81(s); 0,865(s) 0,89-(d, J = 7Hz); 0,90 (d, J = 7Hz); 0,94(s); 0,96(s); 0,965-(s); 0,97(s); 0,98(s); 1,02(s); 1,08 (d, J = 7Hz) 1,09 (d, J = 7Hz): Total 18 H

MS (m/e): 234 ($M^+$), 219, 201, 191, 177, 163, 147, 135, 121, 109

Geruch: moschus-artig, leicht sandelholz-artig, fruchtig, animalisch.

Beispiel 2

A. Blumige Parfümerie-Base

| | Gewichtsteile |
|---|---|
| Synthetische Jasmin-Base | 200 |
| Phenyläthylalkohol | 200 |
| Rosen-Base (synthetisch) | 200 |
| Keton-moschus | 50 |
| Isoraldein (Isomethyl-α-ionon) | 50 |
| C-8-Aldehyd 10% in DPG (Dipropylenglykol) | 10 |
| C-9-Aldehyd 10% in DPG | 10 |
| C-10-Aldehyd 10% in DPG | 10 |
| C-11-Aldehyd 10% in DPG | 10 |
| C-12-Aldehyd (Laurin-Aldehyd 10% in DPG | 10 |
| Dipropylenglykol | 240 |
| | 990 |

Der Zusatz von 10 Teilen 3,5,5,6,8,8-Hexamethyl-1,2,3,4,5,6,7,8-octahydro-2-napthalenon verstärkt die Komposition und verleiht ihr insbesondere eine elegante Moschusnote, die sowohl auf dem frisch getauchten Riechstreifen als auch nach 24 Stunden noch deutlich wahrnehmbar ist.

Die Verbindung (I) eignet sich dementsprechend auch besonders für Damenlinien und zur Parfümierung von kosmetischen Produkten. Sie kann aufgrund ihrer ausserordentlichen Geruchsstärke auch verdünnter eingesetzt werden, in obiger Komposition z.B. genügen auch 5 Teile I.

Die Zugabe von 10 Teilen 6,7-Dihydro-1,1,2,3,3-pentamethyl-5(4H)-indanon(US-PS 3,773,836, 3,816,3) bzw. von 10 Teilen Galaxolide R (1,3,4,5,6,7,8-Hexahydro-4,6,6,7,8,8-hexamethyl-cyclopenta-γ-2-benzopyran) zu dieser blumigen Base verändert diese nur wenig, sie wirkt bloss seifiger und staubiger im ersten Fall und leicht fruchtiger im zweiten Fall.

Gibt man zu der blumigen Base 10 Teile Thibetolide R (Cyclopentadeconolid), so wird deren blumiger Aspekt zwar geringfügig unterstrichen, die gewünschte Aenderung der Komposition in Richtung Moschus ist aber nur schwach wahrnehmbar und kommt zudem erst nach mehreren Stunden zum Ausdruck.

B. Fruchtige Parfümerie-Base

| | Gewichtsteile |
|---|---|
| Hexenyl-isobutyrat | 250 |
| Dimethylbenzylcarbinol-acetat | 100 |
| Aethyl-acetylacetat | 20 |
| Citronellyl-acetat | 20 |
| Citronellyl-äthoxalat | 20 |
| Decyl-acetat | 10 |
| Farnesol | 30 |
| | 450 |

Durch Zusatz von 5 Teilen 3,5,5,6,8,8-Hexamethyl-1,2,3,4,5,6,7,8-octahydro-2-naphthalenon wird die obige Komposition start abgerundet und "harmonisiert", der fruchtige Birnencharakter der Base kommt nun besonders gut zur Geltung; die Komposition wirkt weicher und eignet sich insbesondere für die Anwendung in Kosmetika.

Die Zugabe von 5 Teilen 6,7-Dihydro-1,1,2,3,-pentamethyl-5-(4H)-indanon (US-PS 3,773,836, 3,816,350) bzw. von 5 Teilen Galaxolide R (1,3,4,6,7,8-Hexahydro-4,6,6,7,8,8-hexamethyl-cyclopenta-γ-2-benzopyran) bzw. von 5 Teilen Thibetolide R -(Cyclopentadecanolide) zeitigt andererseits nur einen äusserst geringen Effekt in dieser fruchtigen Komposition, d.h. besagte Zugabe ist kaum wahrnehmbar.

**Ansprüche**

1. 3,5,5,6,8,8-Hexamethyl-1,2,3,4,5,6,7,8-octahydro-2-naphthalenon.

2. Riech-und/oder Geschmackstoffkomposition, gekennzeichnet durch einen Gehalt an 3,5,5,6,8,8-Hexamethyl-1,2,3,4,5,6,7,8-octahydro-2-naphthalenon.

3. Verfahren zur Herstellung 3,5,5,6,8,8-Hexamethyl-1,2,3,4,5,6,7,8-octahydro-2-naphthalenon, dadurch gekennzeichnet, dass man 1,1,3,4,4,6-Hexamethyl-1,2,3,4,5,8-hexahydronaphthalin hydroboriert und das Hydroborierungsprodukt zum 3,5,5,6,8,8-Hexamethyl-1,2,3,4,5,6,7,8-octahydro-2-naphthalenon oxidiert.

4. Verwendung von 3,5,5,6,8,8-Hexamethyl-1,2,3,4,5,6,7,8-octahydro-2-naphthalenon als Riech-und/oder Geschmackstoff.